(19) Europäisches Patentamt
European Patent Office
Office européen des brevets

(11) **EP 4 328 231 A1**

(12) **EUROPEAN PATENT APPLICATION**
published in accordance with Art. 153(4) EPC

(43) Date of publication:
**28.02.2024 Bulletin 2024/09**

(21) Application number: **22791721.8**

(22) Date of filing: **19.04.2022**

(51) International Patent Classification (IPC):
**C07F 3/02** (2006.01)   **C07F 7/08** (2006.01)

(52) Cooperative Patent Classification (CPC):
**C07F 3/02; C07F 7/08**

(86) International application number:
**PCT/JP2022/018137**

(87) International publication number:
**WO 2022/224950 (27.10.2022 Gazette 2022/43)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **22.04.2021 JP 2021072427**

(71) Applicant: **Tokuyama Corporation**
**Shunan-shi, Yamaguchi 745-8648 (JP)**

(72) Inventors:
• **NISHIMOTO Ryosuke**
**Shunan-shi, Yamaguchi 745-8648 (JP)**
• **ODA Hiroyuki**
**Shunan-shi, Yamaguchi 745-8648 (JP)**
• **ABIKO Daisuke**
**Shunan-shi, Yamaguchi 745-8648 (JP)**
• **ICHINOSE Wataru**
**Shunan-shi, Yamaguchi 745-8648 (JP)**
• **ISOMURA Takenori**
**Shunan-shi, Yamaguchi 745-8648 (JP)**

(74) Representative: **Hoffmann Eitle**
**Patent- und Rechtsanwälte PartmbB**
**Arabellastraße 30**
**81925 München (DE)**

(54) **METHOD FOR PRODUCING ORGANIC COMPOUND, AND DEVICE FOR PRODUCING ORGANIC COMPOUND**

(57)     Provided is a method for producing an organic compound, said method including a step for repeating an operation in which a liquid containing an organic solvent is passed through a packed column packed with magnesium, a material containing an organic halide being added to the liquid before it is passed through. Further provided is a device for producing an organic compound, said device comprising: a packed column which is packed with magnesium; a liquid passing unit which repeats an operation in which a liquid containing an organic solvent is passed through the packed column; and a material adding unit which adds a material containing an organic halide to the liquid before it is passed through.

FIG. 1

## Description

TECHNICAL FIELD

The present invention relates to a method for producing an organic compound and a device for producing an organic compound.

BACKGROUND ART

An organomagnesium halide is an organometallic compound used in a Grignard reaction. The Grignard reaction is widely used for synthesis of various organic compounds as a carbon-carbon bond formation reaction (see, for example, Patent Documents 1 and 2). In general, an organomagnesium halide has a high reaction activity but has a low stability.

As a method for producing an organomagnesium halide, a batch-type production method is known in which magnesium is dispersed in an organic solvent and then a solution containing an organic halide is added dropwise to produce an organomagnesium halide (see, for example, Patent Documents 1 and 2). Here, the reaction between the organic halide and magnesium is a solid-liquid reaction, and thus magnesium having an average particle size of about 2 mm or less is used to improve a reaction speed.

Patent Document 1: Japanese Unexamined Patent Application, Publication No.2009-114166
Patent Document 2: Japanese Patent No.3779452

DISCLOSURE OF THE INVENTION

Problems to be Solved by the Invention

However, the reaction between the organic halide and magnesium is accompanied by a very large amount of generated heat, and thus there has been a problem in that the temperature of the reaction system rises, which makes it difficult to control the reaction. In addition, when magnesium remains in the reaction system, the magnesium can cause a side reaction in the subsequent reaction. Thus, there has been a problem in that an excessive amount of the organic halide is used and thus a conversion rate of the organic halide is reduced.

The present invention is directed to provide a method for producing an organic compound and a device for producing an organic compound, which can suppress temperature rise in a reaction system and improve a conversion rate of an organic halide.

Means for Solving the Problems

According to one aspect of the present invention, there is provided a method for producing an organic compound, the method including a step of repeating an operation in which a liquid containing an organic solvent is passed through a packed column packed with magnesium, a material containing an organic halide being added to the liquid before the liquid is passed through the packed column.

In the method for producing an organic compound, the operation in which the liquid is passed through a single packed column can be repeated.

The material can further include a ketone compound or a silane compound.

In the method for producing an organic compound, when a total amount of the organic halide to be added to the liquid before the liquid is passed through the packed column is represented by A [mol] and an amount of magnesium packed in the packed column is represented by B [mol], the number of times of repeating the operation can be determined by a formula

$$A/B \times \alpha$$

where $\alpha$ is 0.9 or more and 1.2.

According to another aspect of the present invention, there is provided a device for producing an organic compound, the device including: a packed column packed with magnesium; a liquid passing unit which repeats an operation in which a liquid containing an organic solvent is passed through the packed column; and a material adding unit which adds a material containing an organic halide to the liquid before the liquid is passed through the packed column.

The liquid passing unit can repeat the operation in which the liquid is passed through a single packed column.

The material can further include a ketone compound or a silane compound.

Effects of the Invention

According to embodiments of the present invention, it is possible to provide a method for producing an organic compound and a device for producing an organic compound which can suppress temperature rise in a reaction system and improve a conversion rate of an organic halide.

BRIEF DESCRIPTION OF THE DRAWINGS

FIG. 1 is a diagram illustrating an example of a device for producing an organic compound according to a present embodiment.
FIG. 2 is a diagram illustrating another example of the device for producing an organic compound according to the present embodiment.

PREFERRED MODE FOR CARRYING OUT THE INVENTION

Hereinafter, embodiments of the present invention will be described.

[Method for Producing Organic Compound]

A method for producing an organic compound according to a present embodiment includes a step of repeating an operation in which a liquid containing an organic solvent is passed through a packed column packed with magnesium (hereinafter referred to as a liquid passing operation), and a material containing an organic halide is added to the liquid before the liquid is passed through the packed column. At this time, magnesium reacts with the organic halide to generate an organomagnesium halide.

When the method for producing an organic compound according to the present embodiment is used, the temperature of the reaction system can be controlled by adjusting a rate at which the material containing an organic halide is added to the liquid before the liquid is passed through the packed column. This suppresses an increase in the temperature of the reaction system. In addition, when a rate of adding the material containing an organic halide to the liquid before the liquid is passed through the packed column is adjusted in accordance with an amount of magnesium packed in the packed column through which the liquid passes and the temperature of the liquid, almost all the organic halides can be reacted. Thus, the conversion rate of the organic halide is improved.

In the present description and claims, the organic compound includes an organometallic compound. Repeating the liquid passing operation for the packed column includes performing the liquid passing operation two or more times for a single packed column, performing the liquid passing operation once for each of a plurality of packed columns, and performing the liquid passing operation two or more times for each of a plurality of packed columns.

In the method for producing an organic compound according to the present embodiment, from the viewpoint of simplification of the device, it is preferable to repeat the liquid passing operation for a single packed column.

Note that in a case where the liquid passing operation is repeated for a plurality of packed columns, the material is added to the liquid before the liquid is passed through the packed column in each packed column.

Alternatively, when the material is added to the liquid before the liquid is passed through the packed column, the material can be added continuously or intermittently.

In a case where the material is a solid, the material is dissolved in an organic solvent and added to the liquid before the liquid is passed through the packed column. On the other hand, in a case where the material is a liquid, the material can be added alone to the liquid before the liquid is passed through the packed column, but the material is preferably dissolved in an organic solvent and added thereto. Here, the organic solvent in which the material is dissolved can be the same as or different from the organic solvent contained in the liquid that passes through the packed column.

The material can further contain a compound that can react with an organomagnesium halide, such as a ketone compound or a silane compound. In a case where the material contains a ketone compound, magnesium and the organic halide react with each other to produce an organomagnesium halide, and then the organomagnesium halide and the ketone compound react with each other to produce a tertiary alcohol. As described above, the temperature rise of the reaction system is suppressed, and the conversion rate of the organic halide is improved. In a case where the material contains a silane compound, magnesium and the organic halide react with each other to produce an organomagnesium halide, and then the organomagnesium halide and the silane compound reacts with each other to produce an organosilicon compound. As described above, the temperature rise of the reaction system is suppressed, and the conversion rate of the organic halide is improved.

Here, an amount of the ketone compound or the silicon compound to be used can be appropriately determined in consideration of reactivity with the organomagnesium halide to be produced. A molar ratio of the ketone compound or the silicon compound to the organic halide is usually 1 or more and 2.5 or less.

In a case where a product in the step of repeating the liquid passing operation for the packed column contains a tertiary alcohol or an organosilicon compound, the method for producing an organic compound according to the present embodiment can further include: a step of adding an acid to decompose an unreacted organomagnesium halide; and a step of purifying the tertiary alcohol or the organosilicon compound.

In a case where the product of the step of repeating the liquid passing operation for the packed column contains an organomagnesium halide, the method for producing an organic compound according to the present embodiment can further include a step of reacting the organomagnesium halide with a compound capable of reacting with the organomagnesium halide, such as a ketone compound or a silane compound.

The packed column only needs to be packed with magnesium in its interior partially or entirely, and to have a shape that allows passing of a liquid, but preferably has a linear structure with a circular cross section and has no branching or bending.

An inside diameter of the packed column is not particularly limited and is, for example, 1 cm or more and 50 cm or less.

A length of the packed column is not particularly limited and is, for example, 30 cm or more and 150 cm or less.

A material of the packed column is not particularly limited, and examples thereof include fluorine resins such as polytetrafluoroethylene, and stainless steel, from the viewpoint of chemical resistance.

An average particle size of magnesium to be packed in the packed column is not particularly limited, and is, for example, 1 mm or longer to 20 mm or shorter.

A shape of the magnesium is not particularly limited, and examples thereof include a pellet shape, a shot shape, a mesh shape, and a rod shape.

A packing ratio of magnesium packed in the packed column is not particularly limited, and is, for example, 30% or more and 99% or less.

A flow rate of the liquid that passes through the column is not particularly limited, and is, for example, 10 ml/min or more and 2000 ml/min or less.

A temperature of the liquid that passes through the column is not particularly limited, and is, for example, -20°C or higher and 100°C or lower.

A rate at which the material is added to the liquid before the liquid is passed through the packed column is not particularly limited, but is, for example, 10 mmol/min or more and 1000 mol/min or less.

The number of times of repeating the liquid passing operation for the packed column is not particularly limited, and is, for example, 2 times or more and 10 times or less.

Note that when the total amount of the organic halide to be added to the liquid before the liquid is passed through the packed column is represented by A [mol] and the amount of magnesium packed in the packed column is represented by B [mol], the number of times of repeating the operation for the packed column can be determined by the formula

$$A/B \times \alpha$$

where $\alpha$ is 0.9 or more and 1.2.

Note that after the liquid passing operation is repeated, the liquid passing operation can be performed for the packed column without adding the material such that the unreacted material is reacted.

[Device for Producing Organic Compound]

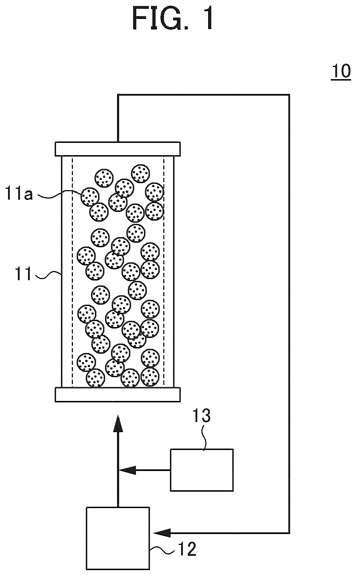

FIG. 1 illustrates an example of a device for producing an organic compound according to a present embodiment.

A device 10 for producing an organic compound has a packed column 11 packed with magnesium 11a, a liquid passing unit 12 that repeats a liquid passing operation for the packed column 11, and a material adding unit 13 that adds a material containing an organic halide to the liquid before the liquid is passed through the column.

The liquid passing unit 12 includes, for example, a buffer tank that holds an organic solvent and a pump that transfers the organic solvent from the buffer tank and repeats the liquid passing operation for the packed column 11. Here, the organic solvent after the liquid is passed through the column returns to the buffer tank, while the organic solvent contains a product and an unreacted material.

The material adding unit 13 includes, for example, a material tank that holds a solution in which an organic halide is dissolved in an organic solvent, and a pump that transfers the solution from the material tank and adds the solution to the liquid before the liquid is passed through the packed column.

Here, instead of the solution in which the organic halide is dissolved in the organic solvent, a solution in which the organic halide and a ketone compound are dissolved in the organic solvent or a solution in which the organic halide and a silane compound are dissolved in the organic solvent can be used.

The device 10 for producing an organic compound can further have a liquid cooling unit (for example, a heat exchanger) between the packed column 11 and the liquid passing unit 12 for cooling the liquid after the liquid is passed through the column.

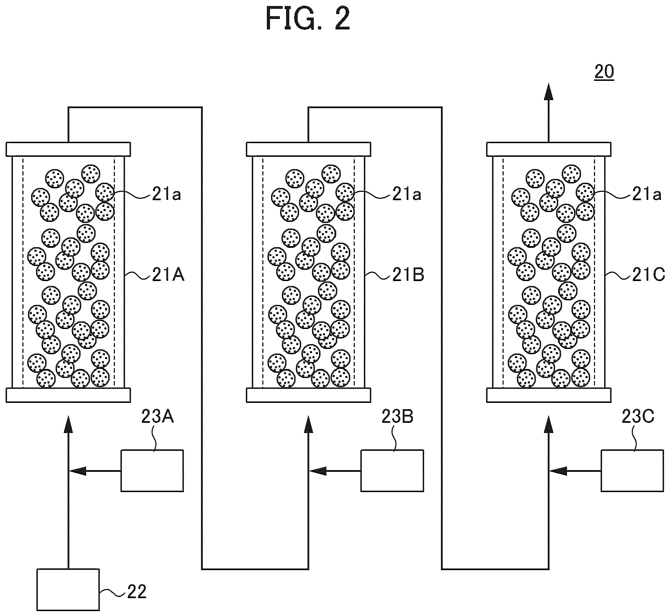

FIG. 2 illustrates another example of the device for producing an organic compound according to the present embodiment.

A device 20 for producing an organic compound has packed columns 21A, 21B, and 21C in which magnesium 21a is packed, a liquid passing unit 22 that repeats a liquid passing operation for the packed columns 21A, 21B, and 21C, and material adding units 23A, 23B, and 23C that add a material containing an organic halide, in the packed columns 21A, 21B, and 21C, to a liquid before the liquid is passed through the column, respectively.

The packed columns 21A, 21B, and 21C are similar to the packed column 11.

The liquid passing unit 22 is the same as the liquid passing unit 12 except that the organic solvent after the liquid is passed through the packed column does not return to the buffer tank.

The material adding units 23A, 23B, and 23C are the same as the material adding unit 13.

The device 20 for producing an organic compound can further have liquid cooling units (for example, heat exchangers) between the packed columns 21A and 21B and between the packed columns 21B and 21C, for cooling the liquid after the liquid is passed through the packed column.

[Organic halide]

The organic halide is not particularly limited as long as it can react with magnesium, and examples thereof include organochlorides, organic bromides, and organic iodides.

Examples of the organic halide include: an alkyl halide; an alkenyl halide; an aryl halide such as chlorobenzene, $\alpha$-chlorotoluene, bromobenzene, $\alpha$-bromotoluene, iodobenzene, and $\alpha$-iodotoluene; an alkylene dihalide; and an arylene dihalide such as o-dichlorobenzene, m-dichlorobenzene, p-dichlorobenzene, o-dibromobenzene, m-dibromobenzene, p-dibromobenzene, o-diiodobenzene, m-diiodobenzene, and p-diiodobenzene.

Examples of the alkyl group in the alkyl halide include a linear or branched alkyl group having 1 to 8 carbon atoms.

Specific examples of the alkyl halide include chloromethane, chloroethane, chloropropane, 2-chloropropane, 1-chloro-2-methylpropane, 2-chloro-2-methylpropane, 2-bromo-2-methylpropane, chlorobutane, bromobutane, chloropentane, chlorocyclopentane, chlorohexane, bromomethane, bromoethane, bromopropane, 2-bromopropane, 1-bromo-2-methylpropane, bromobutane, bromopentane, bromocyclopentane, bromohexane, iodomethane, iodoethane, iodopropane, 2-iodopropane, 1-iodo-2-methylpropane, 2-iodo-2-methylpropane, iodopentane, iodocyclopentane, and iodohexane.

Examples of the alkenyl group in the alkenyl halide include a linear or branched alkenyl group having 2 to 8 carbon atoms.

Specific examples of the alkenyl halide include chloroethylene, 3-chloro-1-propene, bromoethylene, 3-bromo-1-propene, iodoethylene, and 3-iodo-1-propene.

Examples of the alkylene group in the alkylene dihalide include a linear or branched alkylene group having 1 to 8 carbon atoms.

Specific examples of the alkylene dihalide include 1,3-dichloropropane, 1,4-dichlorobutane, 1,5-dichloropentane, 1,3-dibromopropane, 1,4-dibromobutane, 1,5-dibromopentane, 1,3-diiodopropane, 1,4-diiodobutane, and 1,5-diiodopentane.

Among the organic halides, alkyl halides and alkylene dihalides are preferred, and alkyl bromides and alkylene dibromides are more preferred, because they are useful as Grignard reagents.

[Organic Solvent]

The organic solvent is not particularly limited as long as it can dissolve the material, and examples thereof include an ether-based solvent.

Specific examples of the ether-based solvent include diethyl ether, diisopropyl ether, dibutyl ether, tert-butyl methyl ether, 1,2-dimethoxyethane, tetrahydrofuran, 2-methyltetrahydrofuran, and 1,4-dioxane, and two or more types of these compounds can be used in combination. Among these, tetrahydrofuran is preferable from the viewpoint of industrial availability and a high boiling point.

Note that the organomagnesium halide reacts with water and is deactivated, and thus a water content in the organic solvent is preferably less than 500 ppm, and more preferably less than 100 ppm.

The amount of the organic solvent used can be appropriately determined in consideration of a scale of production equipment, a heat removal efficiency, and the like.

[Ketone Compound]

The ketone compound is not particularly limited as long as it can react with an organomagnesium halide. Examples thereof include acetone, methyl ethyl ketone, diethyl ketone, methyl propyl ketone, ethyl propyl ketone, dipropyl ketone, methyl butyl ketone, ethyl butyl ketone, propyl butyl ketone, dibutyl ketone, methyl isopropyl ketone, ethyl isopropyl ketone, diisopropyl ketone, methyl isobutyl ketone, ethyl isobutyl ketone, diisobutyl ketone, propyl isobutyl ketone, methyl vinyl ketone, cyclohexanone, 2-methylcyclopentanone, acetophenone, and benzophenone, and two or more types of these compounds can be used in combination..

[Silane Compound]

The silane compound is not particularly limited as long as it can react with an organomagnesium halide. Examples thereof include: chlorosilane compounds such as dimethyldichlorosilane, methyltrichlorosilane, trimethylchlorosilane, methyldichlorosilane, vinyltrichlorosilane, phenyltrichlorosilane, and trichlorosilane; and alkoxysilane compounds such as methyltrimethoxysilane, dimethyldimethoxysilane, phenyltrimethoxysilane, methyltriethoxysilane, dimethyldiethoxysilane, phenyltriethoxysilane, n-propyltrimethoxysilane, n-propyltriethoxysilane, hexyltrimethoxysilane, hexyltriethoxysilane, and octyltriethoxysilane, and two or more types of these compounds can be used in combination.

EXAMPLES

Hereinafter, examples of the present invention will be described, but the present invention is not limited to the examples.

[Examples 1-1 to 1-3]

The device 10 for producing an organic compound (see FIG. 1) in which a heat exchanger is provided between the packed column 11 and the liquid passing unit 12 was used to produce propyl magnesium bromide.

The packed column having a cylindrical shape, an inside diameter of 2.2 cm, and a height of 100 cm was packed with 350 g of magnesium pellets having an average particle size of 6 mm to give a packing ratio of 80%.

Here, as a method for defining the particle size of magnesium, a major axis length in diameter of three dimensions was adopted. The major axis length in the diameter of three dimensions is a method for defining a particle size, in which, when a rectangular parallelepiped circumscribing an irregular-shaped particle is assumed, a length of the longest side of the three sides in this rectangular parallelepiped is represented as a particle size (for example, see Junichiro Tsubaki, Michitaka Suzuki, Yoshiteru Kanda; Introduction to Grain and Powder Technology, 2nd revised edition, Nikkan Kogyo Shimbun, Ltd. (2016)). In measuring the major axis length of a particle, optical microscopic observation was performed at an observation magnification of 10 times to 200 times, and the number average of major axis lengths of 50 randomly selected particles was defined as the average particle size.

A predetermined amount of 1-bromopropane (BP) was dissolved in a predetermined amount of tetrahydrofuran (THF) (water content of 10 ppm) to obtain a material solution.

Into the buffer tank of the liquid passing unit 12, 5 L of tetrahydrofuran (water content of 10 ppm) was placed, and then the tetrahydrofuran was circulated at a flow rate of 400 ml/min for 30 minutes using a plunger pump having a liquid contact part made of polytetrafluoroethylene while heating the buffer tank and the line to 40°C. Next, after the material solution was placed in the material tank of the material adding unit 13, the material solution was continuously charged to the circulating tetrahydrofuran at a predetermined flow rate for a predetermined time using a plunger pump having a liquid contact part made of polytetrafluoroethylene. At this time, the heat exchanger was used to cool the tetrahydrofuran after the liquid was passed through the packed column to 40°C. The packed column 11, the liquid passing unit 12, the material adding unit 13, and the heat exchanger were connected via a 1/4 inch PFA tube. After charging of the material solution was started, temperatures at an inlet, an intermediate point, and an outlet of the packed column 11 were measured using a K-type thermocouple. After the tetrahydrofuran was circulated for 20 minutes after completion of the charging of the material solution, the entire amount of the circulated tetrahydrofuran was recovered in the buffer tank, propyl magnesium bromide was quantified by gas chromatography analysis, and a conversion rate of 1-bromopropane was calculated.

Table 1 shows evaluation results of the temperature of the packed column and the conversion rate of 1-bromopropane.

[Table 1]

| Example 1 | Composition of material solution | | | Charging condition of material solution | | Temperature of packed column [°C] | | | Conversion rate of BP [%] |
|---|---|---|---|---|---|---|---|---|---|
| | BP [g] | THF [g] | Concentration of BP [mol/L] | Flow rate [ml/min] | Time [min] | Inlet | Intermediate point | Outlet | |
| 1 | 340 | 430 | 3.0 | 7 | 100 | 40 | 53 | 62 | 92 |
| 2 | 150 | 430 | 1.3 | 16 | 40 | 40 | 47 | 58 | 30 |
| 3 | 500 | 430 | 4.4 | 4 | 190 | 40 | 56 | 65 | 86 |

From Table 1, it can be seen that in Examples 1-1 to 1-3, temperature rise of the reaction system is suppressed, and the conversion rate of 1-bromopropane is high.

[Examples 2-1, 2-2]

1,3-bis(dimethylchlorosilyl)propane was produced in the same manner as in Examples 1-1 to 1-3 except that a solution in which 1,3-dibromopropane (DBP) and dichlorodimethylsilane (CMS) were dissolved in tetrahydrofuran at a concentration of 2.8 mol/L, respectively, was used as a material solution, and the material solution was continuously charged into the circulating tetrahydrofuran at a predetermined flow rate for a predetermined time.

Note that a product was identified by $^{1}$H-NMR analysis and $^{29}$Si-NMR analysis. In addition, 1,3-bis(dimethylchlorosilyl)propane was quantified by an internal standard method (internal standard substance: toluene), and a conversion rate of 1,3-dibromopropane was calculated.

Table 2 shows evaluation results of the temperature of the packed column and the conversion rate of 1,3-dibromopropane.

[Table 2]

| Example 2 | Composition of material solution | | Charging condition of material solution | | Temperature of packed column [°C] | | | Conversion rate of DBP [%] |
|---|---|---|---|---|---|---|---|---|
| | Concentration of DBP [mol/L] | Concentration of CMS [mol/L] | Flow rate [ml/min] | Time [min] | Inlet | Intermediate point | Outlet | |
| 1 | 2.8 | 2.8 | 8 | 120 | 40 | 54 | 64 | 89 |
| 2 | 2.8 | 2.8 | 4 | 245 | 40 | 59 | 53 | 93 |

From Table 2, it can be seen that in Examples 2-1 and 2-2, temperature rise of the reaction system is suppressed and the conversion rate of 1,3-dibromopropane is high.

EXPLANATION OF REFERENCE NUMERALS

10, 20 Device for producing organic compound

11 21A, 21B, 21C Packed column

11a, 21a Magnesium

12, 22 Liquid passing unit

13, 23A, 23B, 23C Material adding unit

**Claims**

1. A method for producing an organic compound, the method comprising a step of repeating an operation in which a liquid containing an organic solvent is passed through a packed column packed with magnesium,
   a material containing an organic halide being added to the liquid before the liquid is passed through the packed column.

2. The method for producing an organic compound according to claim 1, wherein the operation in which the liquid is passed through a single packed column is repeated.

3. The method for producing an organic compound according to claim 1 or 2, wherein the material further contains a ketone compound or a silane compound.

4. The method for producing an organic compound according to any one of claims 1 to 3, wherein

   when a total amount of the organic halide to be added to the liquid before the liquid is passed through the packed column is represented by A [mol] and an amount of magnesium packed in the packed column is represented by B [mol], the number of repeating the operation is determined by the formula

   $$A/B \times \alpha$$

   where $\alpha$ is 0.9 or more and 1.2.

5. A device for producing an organic compound, the device comprising:

   a packed column packed with magnesium;
   a liquid passing unit which repeats an operation in which a liquid containing an organic solvent is passed through the packed column; and
   a material adding unit which adds a material containing an organic halide to the liquid before the liquid is passed through the packed column.

6. The device for producing an organic compound according to claim 5, wherein the liquid passing unit repeats an operation in which the liquid is passed through a single packed column.

7. The device for producing an organic compound according to claim 5 or 6, wherein the material further contains a ketone compound or a silane compound.

# FIG. 1

# FIG. 2

## INTERNATIONAL SEARCH REPORT

| International application No. |
|---|
| **PCT/JP2022/018137** |

**A.    CLASSIFICATION OF SUBJECT MATTER**

*C07F 3/02*(2006.01)i; *C07F 7/08*(2006.01)i
FI:   C07F3/02 B; C07F7/08 W

According to International Patent Classification (IPC) or to both national classification and IPC

**B.    FIELDS SEARCHED**

Minimum documentation searched (classification system followed by classification symbols)

C07F3/02; C07F7/08

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C.    DOCUMENTS CONSIDERED TO BE RELEVANT**

| Category* | Citation of document, with indication, where appropriate, of the relevant passages | Relevant to claim No. |
|---|---|---|
| X | CN 102603775 A (SHANGHAI HEGNO MEDICAL DEVELOPMENT CO., LTD.) 25 July 2012 (2012-07-25)<br>claims, paragraphs [0018]-[0077], fig. 1 | 1-2, 5-6 |
| Y | claims, paragraphs [0018]-[0077], fig. 1 | 3-4, 7 |
| X | CN 110590499 A (TAIZHOU BAILLY CHEMICAL CO., LTD.) 20 December 2019 (2019-12-20)<br>claims, paragraphs [0011], [0037], examples, fig. 1 | 1-2, 5-6 |
| Y | claims, paragraphs [0011], [0037], examples, fig. 1 | 3-4, 7 |
| X | JP 2016-525504 A (DPX HOLDINGS B.V.) 25 August 2016 (2016-08-25)<br>claims, paragraphs [0014]-[0060], fig. 1-3 | 1-2, 5-6 |
| Y | claims, paragraphs [0014]-[0060], fig. 1-3 | 3-4, 7 |
| X | JP 52-057148 A (OXON ITALIA SPA) 11 May 1977 (1977-05-11)<br>claims, figures | 1-2, 5-6 |
| Y | claims, figures | 3-4, 7 |

| ☑ Further documents are listed in the continuation of Box C. | ☑ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **06 June 2022** | **14 June 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**

International application No.

**PCT/JP2022/018137**

## C.    DOCUMENTS CONSIDERED TO BE RELEVANT

| Category* | Citation of document, with indication, where appropriate, of the relevant passages | Relevant to claim No. |
|---|---|---|
| Y | JP 2007-290973 A (UNIV NAGOYA) 08 November 2007 (2007-11-08)<br>examples | 3-4, 7 |
| Y | JP 2000-044581 A (DOW CORNING CORP) 15 February 2000 (2000-02-15)<br>claims, examples | 3-4, 7 |
| P, X | WO 2021/153422 A1 (TOKUYAMA CORP) 05 August 2021 (2021-08-05)<br>entire text, all drawings | 1-7 |

Form PCT/ISA/210 (second sheet) (January 2015)

**INTERNATIONAL SEARCH REPORT**
Information on patent family members

International application No.

**PCT/JP2022/018137**

| Patent document cited in search report | | | Publication date (day/month/year) | Patent family member(s) | | | Publication date (day/month/year) |
|---|---|---|---|---|---|---|---|
| CN | 102603775 | A | 25 July 2012 | (Family: none) | | | |
| CN | 110590499 | A | 20 December 2019 | (Family: none) | | | |
| JP | 2016-525504 | A | 25 August 2016 | US | 2016/0137669 | A1 | |
| | | | | claims, paragraphs [0019]-[0109], fig. 1-3 | | | |
| | | | | WO | 2014/207206 | A1 | |
| | | | | EP | 3013836 | A1 | |
| | | | | CN | 105377858 | A | |
| JP | 52-057148 | A | 11 May 1977 | US | 4105703 | A | |
| | | | | claims, figures | | | |
| JP | 2007-290973 | A | 08 November 2007 | (Family: none) | | | |
| JP | 2000-044581 | A | 15 February 2000 | US | 5872274 | A | |
| | | | | claims, examples | | | |
| | | | | EP | 963992 | A2 | |
| WO | 2021/153422 | A1 | 05 August 2021 | TW | 202138376 | A | |
| | | | | entire text, all drawings | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2009114166 A

- JP 3779452 B

**Non-patent literature cited in the description**

- **JUNICHIRO TSUBAKI ; MICHITAKA SUZUKI ; YOSHITERU KANDA.** Introduction to Grain and Powder Technology. Nikkan Kogyo Shimbun, Ltd, 2016